# FASCICULE DE BREVET EUROPEEN

(11) **EP 1 613 376 B1**
(45) Date de publication et mention de la délivrance du brevet: **27.06.2007**
(21) Numéro de dépôt: 04742505.3
(22) Date de dépôt: 15.04.2004
(51) Int. Cl.: A61M 5/30, A61J 1/06

(54) **DISPOSITIF DE LIAISON D'UN RESERVOIR DE PRINCIPE ACTIF SUR UNE BUSE D'INJECTION DANS UN DISPOSITIF D'INJECTION DUDIT PRINCIPE ACTIF**
VORRICHTUNG ZUR VERBINDUNG EINES WIRKSTOFFBEHÄLTERS AUF EINER INJEKTIONSDÜSE IN EINEM INJEKTIONSGERÄT
DEVICE FOR CONNECTING AN ACTIVE SUBSTANCE CONTAINER TO AN INJECTION NOZZLE IN A DEVICE USED TO INJECT SAID ACTIVE SUBSTANCE

(30) Priorité: 16.04.2003 FR 0304762
(43) Date de publication de la demande: 11.01.2006
(73) Titulaire: Crossject, 75004 Paris (FR)
(72) Inventeur: ALEXANDRE, Patrick, F-70100 Gray (FR); BAUD, Georges, F-83260 La Crau (FR); BROUQUIERES, Bernard, F-83100 Toulon (FR)
(74) Mandataire: Maureau, Philippe
(86) Numéro de dépôt international: PCT/FR2004/000923
(87) Numéro de publication internationale: WO 2004/093947

(56) Documents cités:
- WO-A-01/30419
- US-A- 4 479 801
- US-A- 5 919 159
- US-A- 5 989 226
- US-A- 6 132 395

## Description

Le domaine technique de l'invention est celui des dispositifs d'injection sans aiguille, préremplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet n°FR 2 815 544 (équivalente à WO 02/34317), un ensemble mécanique et un ensemble pharmaceutique. L'ensemble mécanique se compose notamment d'un mécanisme d'actionnement du dispositif et d'une source d'énergie, constituée par exemple d'un générateur pyrotechnique de gaz, destinée à provoquer l'injection du principe actif à travers la peau du patient. L'ensemble pharmaceutique comporte le principe actif liquide. Le principe actif est en général placé dans un tube destiné à être inséré ensuite dans l'ensemble mécanique. Les deux ensembles sont en général assemblés séparément, le remplissage du tube par le principe actif devant être parfaitement contrôlé et effectué dans un endroit confiné pour éviter que des impuretés ne s'infiltrent dans le tube et viennent polluer le principe actif. Il est notamment nécessaire qu'aucune impureté ne vienne se déposer dans les zones par lesquelles le principe actif doit s'écouler. Pour éviter l'infiltration de ces impuretés dans le tube, le tube peut être assemblé lors de la fabrication de l'ensemble pharmaceutique sur la buse d'injection du principe actif et plaqué contre celle-ci pour créer une liaison étanche entre les deux éléments. Les deux éléments, c'est-à-dire le tube rempli par le principe actif et la buse d'injection constituent l'ensemble pharmaceutique. Après l'assemblage des deux éléments de l'ensemble pharmaceutique et le remplissage du tube par le principe actif et avant l'assemblage de l'ensemble pharmaceutique sur l'ensemble mécanique, il peut s'écouler une certaine durée pendant laquelle l'ensemble pharmaceutique est transporté et stocké dans un environnement non confiné, comme par exemple un entrepôt. Pendant cette durée, il est donc nécessaire de conserver une parfaite liaison entre la buse d'injection et le tube et un bon plaquage du tube contre la buse d'injection afin d'éviter la pénétration d'impuretés dans le tube. Afin de conserver une parfaite liaison entre la buse et le tube, il est nécessaire de ne pas trop solliciter mécaniquement la buse lors de sa fixation sur le tube.

Il est connu du brevet US 6,132,395 un dispositif d'injection sans aiguille comportant un réservoir et une buse en polycarbonate destinée à être fixée sur le réservoir constitué d'un tube en verre. La buse est située à une extrémité du tube et comporte plus particulièrement quatre pattes s'étendant sur toute la longueur du tube et se terminant par des bossages permettant le clipsage de la buse sur le tube. Les pattes forment ainsi une griffe venant enserrer le tube pour fixer la buse. Dans ce brevet, la buse est fixée directement grâce à ses pattes. Les pattes effectuent une flexion vers l'extérieur sur toute la longueur du tube jusqu'à ce que le tube soit complètement enserré. Lors du clipsage de la buse sur le tube, cette sollicitation mécanique unique des pattes peut créer des déformations irréversibles des pattes si celles-ci sont dans un matériau dur comme par exemple le polycarbonate et ainsi entraîner une mauvaise liaison entre le tube et la buse.

Un dispositif d'injection selon la préambule de la revendication 1 est connu du brevet US 5,919,159.

Le but de l'invention est donc de conserver une liaison parfaitement étanche entre le tube et la buse d'injection pour éviter la pénétration d'impuretés dans le tube lorsque l'ensemble pharmaceutique n'est pas encore assemblé sur l'ensemble mécanique pour fabriquer le dispositif d'injection sans aiguille complet.

Ce but est atteint par un dispositif d'injection d'un principe actif à travers la peau d'un patient comprenant notamment une buse d'injection et un tube en verre destiné à recevoir ledit principe actif à injecter, ledit tube venant se fixer sur ladite buse à l'aide de moyens de liaison, les moyens de liaison comportant au moins trois bossages identiques et solidaires de la buse, lesdits bossages comportant chacun une partie inclinée terminée par un épaulement, ledit épaulement coopérant avec une collerette formée sur le tube et située à l'une des extrémités du tube, ladite collerette servant de butée anti-retour pour le tube lorsque celui-ci est relié à la buse, ledit dispositif étant caractérisé en ce que les bossages sont reliés entre eux par des branches de liaison.

Selon une particularité, les branches de liaison ont une hauteur de 1,4 mm. Selon l'invention, l'épaisseur et la hauteur des branches de liaison sont étudiées pour rendre les branches de liaison suffisamment souples pour s'allonger et fléchir au niveau des bossages lors du clipsage du tube. La souplesse des branches de liaison permet notamment à chacun des bossages d'épouser la forme du tube de verre.

Selon une autre particularité, les bossages sont portés par les branches de liaison, lesdites branches de liaison étant solidaires de la buse par des plots de liaison et reliées entre elles pour définir une couronne sensiblement circulaire dont le diamètre est sensiblement égal au diamètre externe de la collerette du tube. Selon l'invention, la géométrie de la couronne servant au clipsage du tube sur la buse a été étudiée pour répartir au mieux les contraintes s'exerçant sur le tube.

Ainsi, grâce à la présence des branches de liaison portant les bossages, la déformation nécessaire au clipsage du tube sur la buse ne se limite pas à une simple flexion au niveau des bossages mais est constituée à la fois d'un allongement des branches de liaison et d'une flexion de ces branches de liaison au niveau de chacun des bossages. Les plots de liaison assurent quant à eux la rigidité et le blocage de la liaison par clipsage entre le tube et la buse.

Selon l'invention, chacun des bossages constitue un point de contact de la buse avec le tube.

Selon une particularité, la partie inclinée des bossages est inclinée en direction de la buse et vers l'intérieur de la buse. Cette partie inclinée permet notamment lors de l'assemblage par clipsage du tube sur la buse, de guider le tube et d'écarter progressivement les bossages pour laisser passer la collerette du tube. Le degré d'inclinaison de cette partie inclinée doit être étudié pour limiter au maximum l'effort nécessaire à l'écartement des bossages lors du passage de la collerette du tube.

Selon une autre particularité, chaque bossage est incurvé, les bossages s'inscrivant tous dans un même cercle dont le diamètre est sensiblement égal au diamètre externe de la collerette du tube. Selon l'invention, l'effort de plaquage du tube contre la buse n'est donc pas réparti, au niveau de chaque bossage, sur un point de contact mais sur une ligne ou même sur une surface.

Selon une autre particularité, l'angle d'ouverture défini par les deux segments joignant le centre du cercle aux extrémités de chaque bossage incurvé est compris entre 17 et 23 degrés. Selon l'invention, pour chaque bossage, plus l'angle défini ci-dessus sera long et plus la répartition des contraintes sur le tube de verre sera bonne.

Selon une autre particularité, les bossages sont régulièrement espacés entre eux. L'espacement des bossages doit en effet être régulier pour obtenir une répartition homogène des contraintes sur le tube.

Selon une autre particularité, la buse présente une surface plane, les bossages étant situés à une distance non nulle de ladite surface, l'espace compris entre l'épaulement des bossages et ladite surface correspondant sensiblement à l'épaisseur de la collerette du tube.

Selon une autre particularité, l'extrémité de chacun des bossages a une forme arrondie. La ligne de contact de chacun des bossages avec le tube est située au sommet de l'arrondi. L'arrondi permet donc de supprimer toute arête vive et donc de limiter les agressions éventuelles sur le tube de verre.

Selon une autre particularité, le diamètre du cercle dans lequel sont inscrits les bossages est de 13.2 mm et la forme arrondie des bossages a un rayon de 0.1 mm. Selon l'invention, plus le diamètre du cercle est grand, plus la liaison du tube sur la buse est stable et plus les efforts exercés sur le tube de verre sont faibles.

Selon un mode de réalisation, chaque bossage est porté par une tige fixée à la buse et apte à se déformer élastiquement.

Selon un mode de réalisation préféré de l'invention, la buse, les bossages, les, branches de liaison et les plots de liaison sont faits d'une même pièce.

Selon une particularité, la pièce constituée de la buse, des bossages, des branches de liaison et des plots de liaison est fabriquée en polycarbonate. Selon l'invention, le matériau constituant cette pièce doit pouvoir répondre à un certain nombre de contraintes. Ce matériau devra notamment être suffisamment dur pour être traversé par le principe actif liquide sans se détériorer et être suffisamment souple pour pouvoir venir clipser le tube. De plus, ce matériau devra être agréé pour son utilisation dans le domaine de la pharmacie et ne pas être agressif vis-à-vis du verre pour ne pas rayer et endommager le tube.

Selon un mode de réalisation préféré de l'invention, le dispositif d'injection est sans aiguille et comporte comme source d'énergie pour l'injection du principe actif, un générateur pyrotechnique de gaz.

L'invention, avec ses caractéristiques et avantages, ressortira plus clairement à la lecture de la description faite en référence aux dessins annexés dans lesquels :
La figure 1 représente, en mode éclaté, un dispositif d'injection sans aiguille selon l'invention:
La figure 2 représente en perspective une buse d'injection utilisée dans le dispositif d'injection selon l'invention.
La figure 3 représente en perspective un réservoir destiné à recevoir le principe actif liquide à injecter.
La figure 4 représente, vue de dessus la buse d'injection représentée en figure 2.
La figure 5 représente une coupe longitudinale suivant A-A de la buse d'injection représentée en figure 4.
La figure 6 représente en perspective le réservoir visible en figure 3 assemblé sur la buse d'injection pour former un ensemble pharmaceutique.
La figure 7 représente une coupe transversale de l'ensemble pharmaceutique visible en figure 6.

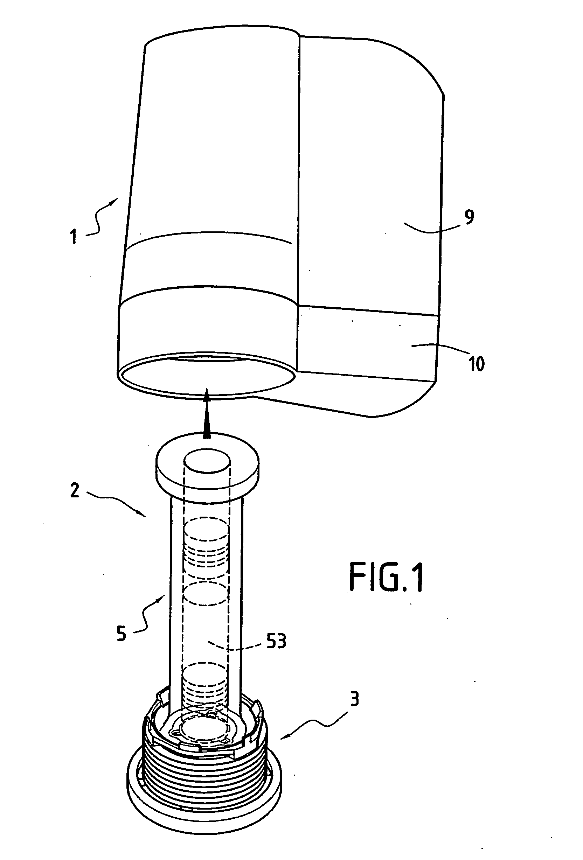

Un dispositif 1 d'injection selon l'invention, tel que représenté en figure 1, est sans aiguille et comporte un corps (non visible) inséré sous un capot 9 d'actionnement du dispositif 1, ce capot étant obturé par un bouchon 10. Le dispositif 1 aura par exemple une forme compacte dont les avantages sont plus particulièrement décrits dans le brevet n° FR 2 815 544. L'actionnement d'un tel dispositif 1 par le patient, à l'aide du capot 9, est également décrit dans le brevet FR 2 815 544. Lors du processus d'assemblage du dispositif 1, le corps est destiné à recevoir une pluralité d'éléments. Ainsi, une fois assemblé, le corps comporte ou délimite successivement, de l'amont vers l'aval, un dispositif d'initiation comme par exemple un dispositif de percussion, une amorce, une charge pyrotechnique, ces trois éléments formant un générateur de gaz, une chambre de combustion, un réservoir 5 contenant un principe actif liquide à injecter et un système d'injection.

Selon l'invention, le dispositif 1 d'injection sans aiguille comporte deux ensembles principaux, un ensemble mécanique et un ensemble 2 pharmaceutique. Ces deux ensembles sont distingués car, en règle générale, ils sont assemblés séparément, l'assemblage de l'ensemble 2 pharmaceutique nécessitant un contrôle spécifique et une attention toute particulière notamment en ce qui concerne le remplissage du principe actif 53.

L'ensemble mécanique comporte notamment le générateur de gaz et le mécanisme d'actionnement du dispositif selon l'invention. L'ensemble 2 pharmaceutique comporte le réservoir 5 de principe actif 53 et le système d'injection du principe actif 53.

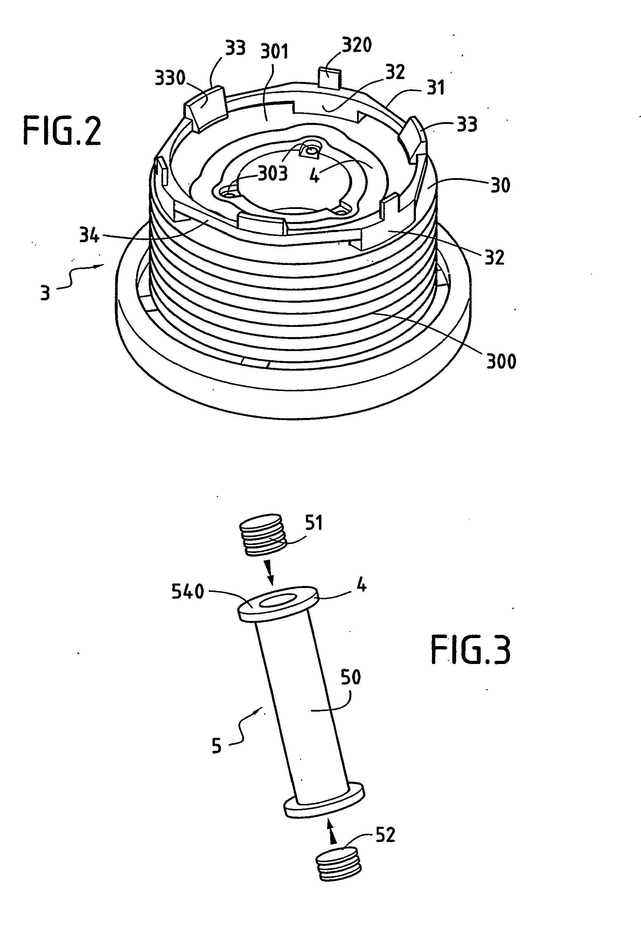

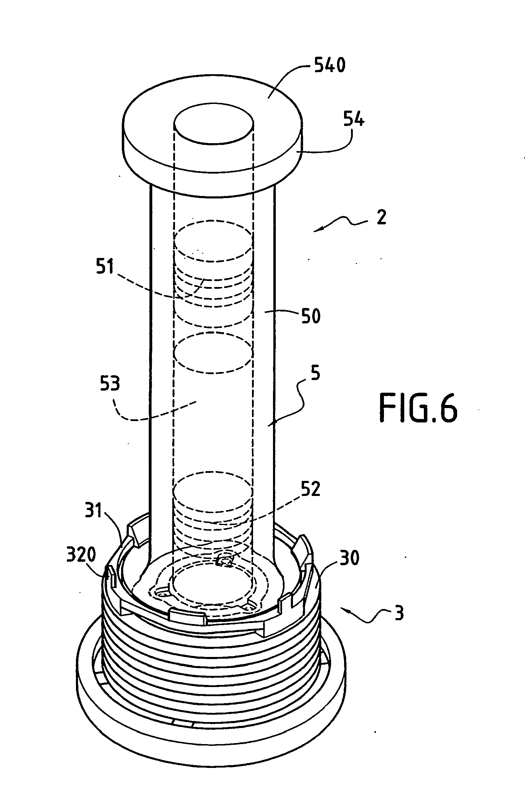

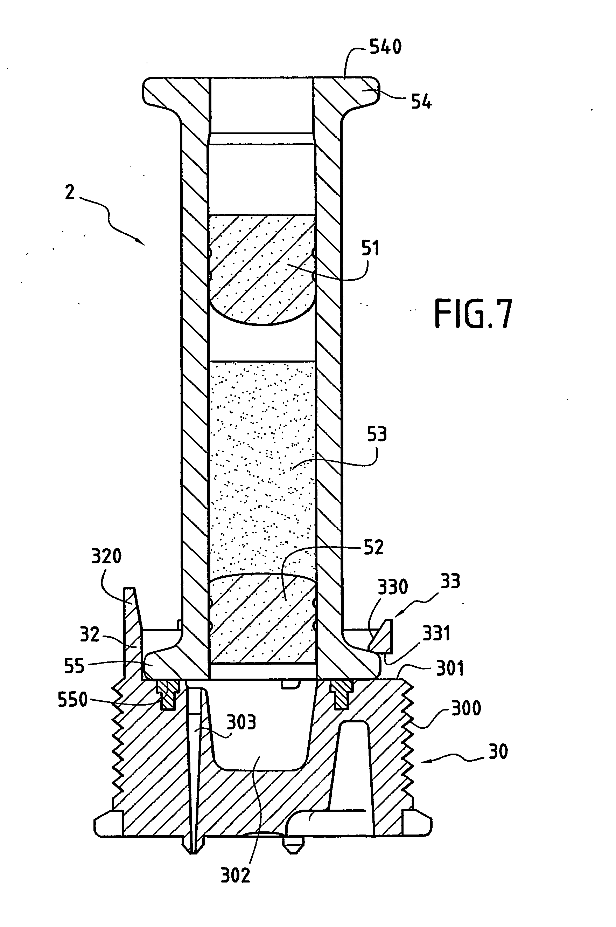

Le réservoir 5 destiné à recevoir le principe actif 53 est représenté plus précisément en figure 3. Il est par exemple constitué d'un tube 50 en verre ouvert à ses deux extrémités. Le tube 50 comporte une collerette 54,55 à chacune de ses extrémités. Chaque collerette 54,55 définit à l'extrémité du tube 50 une surface 540,550 annulaire plane. Le tube 50 est inséré dans le corps du dispositif 1 de manière à être relié, à son extrémité la plus en amont, à la chambre de combustion du dispositif 1 et à son extrémité la plus en aval au système d'injection. Le principe actif 53 (figures 6 et 7) est par exemple emprisonné dans le tube 50 en verre entre un bouchon-piston amont 51 et un bouchon-piston aval 52 enfoncés dans le tube 50. Les bouchons-pistons amont 51 et aval 52. sont réalisés par exemple dans un matériau déformable à base d'élastomère.

Selon l'invention, le système d'injection comporte une buse 3 d'injection à travers laquelle est injecté le principe actif 53 contenu dans le réservoir 5. La buse 3 d'injection comporte une pièce 30 cylindrique autour de laquelle est formé un filetage 300 permettant à la buse 3 de venir se visser sur le corps du dispositif 1 d'injection sans aiguille. La pièce 30 cylindrique comporte une surface 301 plane, formant un plan d'appui pour le tube 50, perpendiculaire à l'axe A1 (figure 5) de la buse 3, au milieu de laquelle est formé un trou 302 borgne. La pièce 30 cylindrique est traversée par une pluralité de canaux 303 d'injection par lesquels le liquide s'écoule lors de l'injection. Sur la figure 2, ces canaux 303 sont au nombre de trois. Ils sont formés parallèlement à l'axe A1 de la buse 3, en périphérie du trou 302 borgne formé sur la pièce cylindrique 30. En fin d'injection, le bouchon-piston aval 52 vient se loger dans le trou 302 borgne, ce qui libère le principe actif 53 liquide qui peut alors s'échapper par les canaux 303 périphériques communiquant avec l'intérieur du tube 50.

Selon l'invention, la buse 3 comporte également une couronne 31 d'un diamètre sensiblement égal au diamètre de la pièce 30 cylindrique. Cette couronne 31 a la forme d'un bandeau circulaire disposé de manière coaxial par rapport à l'axe A1 de la buse 3, la surface dudit bandeau étant parallèle à l'axe A1 de la buse 3. Cette couronne 31 est située à une distance déterminée non nulle de la pièce 30 cylindrique et est reliée à celle-ci par l'intermédiaire de plots 32 de liaison se dressant sur la surface de la pièce 30 cylindrique à proximité du bord externe de la pièce 30 cylindrique. Ces plots 32 de liaison sont par exemple au nombre de trois répartis de manière équilibrée entre la couronne 31 et la pièce 30 cylindrique, c'est-à-dire à un angle de 60° les uns par rapport aux autres. De chaque plot émerge une tige 320, chaque tige 320 comportant par exemple une partie inclinée (Figure 5) permettant de guider le tube 50 lors de son assemblage sur la buse 3. A équidistance entre chaque plot 32, c'est-à-dire décalé de 30° par rapport à chacun des plots 32, la couronne 31 comporte un bossage 33. Chaque bossage 33 est allongé et incurvé suivant la courbure de la couronne 31. L'angle défini par les segments joignant le centre de la couronne 31 à chacune des deux extrémités d'un bossage 33 est par exemple compris entre 17° et 23° et est préférentiellement de 20°. Chaque bossage 33 est constitué d'une portion 330 (Figure 2) inclinée par rapport à la surface définie par le bandeau. Cette portion 330 est inclinée vers l'intérieur de la buse 3 et en direction de la pièce 30 cylindrique et se termine par un épaulement 331. Le sommet ou extrémité de chacun des bossages 33, défini comme la surface de liaison entre la portion 330 inclinée et l'épaulement 331, a une forme arrondie 332 (Figure 5), d'un rayon par exemple égal à 0,1 mm. La couronne 31 a par exemple un diamètre de 15 mm. Le diamètre de la couronne 31 au sommet du bossage 33 est par exemple de 13,2 mm. La hauteur du bandeau définissant la couronne est par exemple de 1,4 mm. L'épaisseur de la couronne 31 est plus importante au niveau des plots 32.

Selon l'invention, le tube 50 est destiné à être assemblé, par l'une de ses extrémités, sur la buse 3 pour constituer l'ensemble 2 pharmaceutique. Pour cela, l'une des extrémités du tube 50 est clipsée sur la buse 3. L'espace défini entre la couronne 31 et la pièce 30 cylindrique est sensiblement égal à l'épaisseur de la collerette 55 formée à une extrémité du tube 50. Cet espace est donc suffisant pour loger ladite collerette 55. Le tube 50 est inséré sur la buse 3 de sorte que son axe soit sensiblement confondu avec celui (A1) de la buse 3.

Lors de l'assemblage du tube 50 sur la buse 3, le tube 50 est tout d'abord guidé par les tiges 320 prolongeant les plots 32 de liaison. La collerette 55 située à l'extrémité du tube 50 suit ensuite les portions 330 inclinées des bossages 33. Le degré d'inclinaison des portions 330 inclinées des bossages 33 joue un rôle dans l'effort qu'il est nécessaire de produire pour écarter les bossages 33 et laisser passer la collerette 55. En continuant d'enfoncer le tube 50, la collerette 55 passe au-delà des sommets des bossages 33 et vient se loger sous les bossages 33. La surface 550 annulaire située à l'extrémité du tube 50 est alors maintenue plaquée contre la surface de la pièce cylindrique par l'intermédiaire des bossages 33. Chacun des épaulements 331 des bossages 33 constitue une butée anti-retour au mouvement d'extraction du tube 50 par rapport à la buse 3.

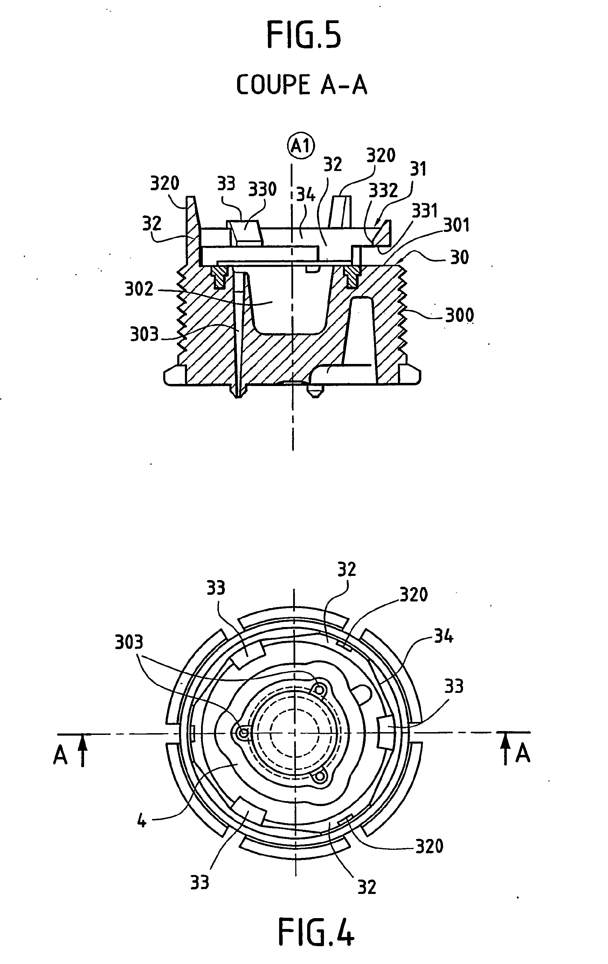

Selon l'invention, les branches 34 (Figure 4) de liaison, définies par les parties de la couronne 31 portant les bossages et reliant les plots 32 de liaison sont d'une épaisseur et d'une raideur déterminée et suffisante pour garantir une certaine flexibilité à la couronne 31 et ainsi écarter suffisamment les bossages 33 pour laisser passer la collerette 55 du tube 50 lors du clipsage du tube 50 sur la buse 3. De plus, les branches 34 de liaison doivent disposer d'une épaisseur et d'une hauteur suffisante pour être suffisamment souples et élastiques et faire en sorte que les bossages 33 viennent épouser la forme du tube 50 de verre.

Selon l'invention, la buse 3 sera par exemple fabriquée d'une seule pièce. Le matériau employé pour la fabrication de la buse 3 devra être d'une raideur suffisante pour garantir le plaquage du tube 50 contre la buse 3. De plus ce matériau ne doit pas être agressif vis-à-vis du verre et être apte à être utilisé en pharmacie. Le polycarbonate est un matériau répondant à ces différents critères.

L'espace séparant la couronne 31 de la pièce 30 cylindrique, dans lequel vient s'insérer la collerette 55, doit être étudié de manière à garantir un plaquage de la surface 550 annulaire, définie à l'extrémité du tube 50, contre la surface 301 de la pièce 30 cylindrique et ainsi une liaison étanche entre le tube 50 et la buse 3.

Selon l'invention, lorsque l'ensemble 2 pharmaceutique est assemblé sur l'ensemble mécanique pour former le dispositif 1 d'injection complet, la liaison étanche entre le tube 50 et la buse 3 est réalisée par la compression d'un joint 4 (figures 2 et 4) intégré dans la buse 3.

Le fonctionnement d'un tel dispositif 1 d'injection sans aiguille ayant des composants tels que ceux définis dans la présente demande est décrit en détail dans la demande de brevet français FR 2 815 544. Le fonctionnement global d'un tel dispositif 1 peut toutefois être résumé de la manière suivante :
Au repos, un percuteur du dispositif de percussion est par exemple en appui contre une butée à l'aide d'un ressort précontraint dont l'axe est sensiblement confondu avec l'axe du percuteur. Une manipulation du patient provoque la libération du percuteur qui, sous l'effet de la détente du ressort, vient percuter l'amorce située dans le même axe. L'initiation de l'amorce entraîne ensuite l'allumage de la charge pyrotechnique du générateur de gaz. Sous l'action des gaz générés par la charge pyrotechnique, le bouchon-piston amont 51 présent dans le tube 50 du réservoir 5 se déplace et pousse à son tour le principe actif 53 en direction du système d'injection. Le bouchon-piston aval 52 est lui-même poussé jusqu'à venir se loger dans le trou 302 borgne de la buse 3 d'injection. Ainsi, la communication entre l'intérieur du tube et les canaux périphériques est effectuée et donc le principe actif 53 peut rejoindre les canaux 303 périphériques et être éjecté hors du dispositif 1.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif (1) d'injection comprenant notamment une buse (3) d'injection et un tube (50) en verre destiné à recevoir un principe actif (53) à injecter, ledit tube (50) venant se fixer sur ladite buse (3) à l'aide de moyens de liaison, les moyens de liaison comportant au moins trois bossages (33) identiques et solidaires de la buse (3), lesdits bossages (33) comportant chacun une partie (330) inclinée terminée par un épaulement (331), ledit épaulement (331) coopérant avec une collerette (55) formée sur le tube (50) et située à l'une des extrémités du tube (50), ladite collerette servant de butée anti-retour pour le tube (50) lorsque celui-ci est relié à la buse (3), ledit dispositif étant **caractérisé en ce que** les bossages (33) sont reliés entre eux par des branches (34) de liaison.

2. Dispositif (1) selon la revendication 1, **caractérisé en ce que** la partie (330) inclinée des bossages (33) est inclinée en direction de la buse (3) et vers l'intérieur de la buse (3).

3. Dispositif (1) selon la revendication 1, **caractérisé en ce que** chaque bossage (33) est incurvé, les bossages (33) s'inscrivant dans un même cercle dont le diamètre est sensiblement égal au diamètre externe de la collerette (55) du tube (50).

4. Dispositif (1) selon la revendication 3, **caractérisé en ce que** l'angle d'ouverture défini par les deux segments joignant le centre du cercle aux extrémités de chaque bossage (33) incurvé est compris entre 17 et 23 degrés.

5. Dispositif (1) selon l'une des revendications 1 à 4, **caractérisé en ce que** les bossages (33) sont régulièrement espacés entre eux.

6. Dispositif (1) selon l'une des revendications 1 à 5, **caractérisé en ce que** la buse (3) présente une surface (301) plane, les bossages (33) étant situés à une distance non nulle de ladite surface (301), l'espace compris entre l'épaulement (331) des bossages (33) et ladite surface (301) correspondant sensiblement à l'épaisseur de la collerette (55) du tube (50).

7. Dispositif (1) selon l'une des revendications 1 à 6, **caractérisé en ce que** l'extrémité de chacun des bossages (33) a une forme arrondie (332).

8. Dispositif (1) selon la revendication 7, **caractérisé en ce que** le diamètre du cercle est de 13,2 mm et **en ce que** la forme arrondie (332) a un rayon de 0,1 mm.

9. Dispositif (1) selon l'une des revendications 1 à 8, **caractérisé en ce que** chaque bossage (33) est porté par une tige fixée à la buse (3) et apte à se déformer élastiquement.

10. Dispositif (1) selon la revendication 1, **caractérisé en ce que** les branches (34) de liaison ont une hauteur de 1,4 mm.

11. Dispositif (1) selon la revendication 1 ou 10, **caractérisé en ce que** les bossages (33) sont portés par les branches (34) de liaison, lesdites branches (34) de liaison étant solidaires de la buse (3) par des plots (32) de liaison et reliées entre elles pour définir une couronne (31) sensiblement circulaire dont le diamètre est sensiblement égal au diamètre externe de la collerette (55) du tube (50).

12. Dispositif (1) selon la revendication 11, **caractérisé en ce que** la buse (3), les bossages (33), les branches (34) de liaison et les plots (32) de liaison sont faits d'une même pièce.

13. Dispositif (1) selon la revendication 12, **caractérisé en ce que** la pièce est fabriquée en polycarbonate.

## Claims

1. Injection device (1) comprising in particular an injection nozzle (3) and a glass tube (50) which is designed to receive an active principle (53) to be injected, the said tube (50) being secured to the said nozzle (3) by means of connection means, the connection means comprising at least three identical bosses (33) which are integral with the nozzle (3), the said bosses (33) each comprising an inclined part (330) which ends in a shoulder (331), the said shoulder (331) co-operating with a flange (55) which is formed on the tube (50) and is situated at one of the ends of the tube (50), the said flange acting as a non-return stop for the tube (50) when the latter is connected to the nozzle (3), the said device being **characterised in that** the bosses (33) are connected to one another by connection branches (34).

2. Device (1) according to claim 1, **characterised in that** the inclined part (330) of the bosses (33) is inclined in the direction of the nozzle (3) and towards the interior of the nozzle (3).

3. Device (1) according to claim 1, **characterised in that** each boss (33) is curved, the bosses (33) being contained in a single circle, the diameter of which is substantially equal to the outer diameter of the flange (55) of the tube (50).

4. Device (1) according to claim 3, **characterised in that** the angle of opening defined by the two segments which join the centre of the circle to the ends of each curved boss (33) is between 17 and 23 degrees.

5. Device (1) according to any one of claims 1 to 4, **characterised in that** the bosses (33) are regularly spaced from one another.

6. Device (1) according to any one of claims 1 to 5, **characterised in that** the nozzle (3) has a flat surface (301), the bosses (33) being situated at a non-zero distance from the said surface (301), the space contained between the shoulder (331) of the bosses (33) and the said surface (301) corresponding substantially to the thickness of the flange (55) of the tube (50).

7. Device (1) according to any one of claims 1 to 6, **characterised in that** the end of each of the bosses (33) has a rounded shape (332).

8. Device (1) according to claim 7, **characterised in that** the diameter of the circle is 13.2 mm and the rounded shape (332) has a radius of 0.1 mm.

9. Device (1) according to any one of claims 1 to 6, **characterised in that** each boss (33) is supported by a rod which is secured to the nozzle (3), and can be resiliently deformed.

10. Device (1) according to claim 1, **characterised in that** the connection branches (34) have a height of 1.4 mm.

11. Device (1) according to claim 1 or claim 10, **characterised in that** the bosses (33) are supported by the connection branches (34), the said connection branches (34) being rendered integral with the nozzle (3) by connection studs (32), and connected to one another in order to define a substantially circular ring (31), the diameter of which is substantially equal to the outer diameter of the flange (55) of the tube (50).

12. Device (1) according to claim 11, **characterised in that** the nozzle (3), the bosses (33), the connection branches (34) and the connection studs (32) are produced in a single piece.

13. Device (1) according to claim 12, **characterised in that** the part is made of polycarbonate.

## Patentansprüche

1. Einspritzvorrichtung (1), die insbesondere eine Einspritzdüse (3) und eine Glasröhre (50) aufweist, die dazu bestimmt ist, einen einzuspritzenden Wirkstoff (53) aufzunehmen, wobei die Röhre (50) an der Düse (3) mit Hilfe von Verbindungsmitteln befestigt wird, wobei die Verbindungsmittel mindestens drei identische Höcker (33) aufweist, die mit der Düse (3) verbunden sind, wobei die Höcker (33) jeweils einen schrägen Teil (330) aufweisen, der in einem Ansatz (331) endet, wobei der Ansatz (331) mit einem Kragen (55) zusammenarbeitet, der auf der Röhre (50) ausgebildet ist und sich an einem der Enden der Röhre (50) befindet, wobei der Kragen als Rücklaufschutzanschlag für die Röhre (50) dient, wenn diese mit der Düse (3) verbunden ist, wobei die Vorrichtung **dadurch gekennzeichnet ist, dass** die Höcker (33) untereinander durch Verbindungszweige (34) verbunden sind.

2. Vorrichtung (1) nach Anspruch 1, **dadurch gekennzeichnet, dass** der schräge Teil (330) der Höcker (33) in Richtung der Düse (3) und zum Inneren der Düse (3) geneigt ist.

3. Vorrichtung (1) nach Anspruch 1, **dadurch gekennzeichnet, dass** jeder Höcker (33) gebogen ist, wobei die Höcker (33) in einen gleichen Kreis fallen, dessen Durchmesser im Wesentlichen gleich dem Außendurchmesser des Kragens (55) der Röhre (50) ist.

4. Vorrichtung (1) nach Anspruch 3, **dadurch gekennzeichnet, dass** der Öffnungswinkel, der von den zwei Segmenten definiert wird, die die Mitte des Kreises mit den Enden jedes gebogenen Höckers (33) verbinden, zwischen 17° und 23° liegt.

5. Vorrichtung (1) nach einem der Ansprüche 1 bis 4, **dadurch gekennzeichnet, dass** die Höcker (33) regelmäßig zwischeneinander beabstandet sind.

6. Vorrichtung (1) nach einem der Ansprüche 1 bis 5, **dadurch gekennzeichnet, dass** die Düse (3) eine ebene Fläche (301) aufweist, wobei sich die Höcker (33) in einer Entfernung nicht gleich Null von der Fläche (301) befinden, wobei der Raum zwischen dem Ansatz (331) der Höcker (33) und der Fläche (301) im Wesentlichen der Stärke des Kragens (55) der Röhre (50) entspricht.

7. Vorrichtung (1) nach einem der Ansprüche 1 bis 6, **dadurch gekennzeichnet, dass** das Ende jedes der Höcker (33) eine gerundete Form (332) hat.

8. Vorrichtung (1) nach Anspruch 7, **dadurch gekennzeichnet, dass** der Durchmesser des Kreises 13,2 mm beträgt, und dass die gerundete Form (332) einen Radius von 0,1 mm hat.

9. Vorrichtung (1) nach einem der Ansprüche 1 bis 8, **dadurch gekennzeichnet, dass** jeder Höcker (33) von einem Schaft getragen ist, der an der Düse (3) befestigt ist und sich elastisch verformen kann.

10. Vorrichtung (1) nach Anspruch 1, **dadurch gekennzeichnet, dass** die Verbindungszweige (34) eine Höhe von 1,4 mm haben.

11. Vorrichtung (1) nach Anspruch 1 oder 10, **dadurch gekennzeichnet, dass** die Höcker (33) von den Verbindungszweigen (34) getragen werden, wobei die Verbindungszweige (34) fest mit der Düse (3) durch Verbindungsklötze (32) verbunden und untereinander verbunden sind, um einen im Wesentlichen kreisförmigen Kranz (31) zu definieren, dessen Durchmesser im Wesentlichen gleich dem Außendurchmesser des Kragens (55) der Röhre (50) ist.

12. Vorrichtung (1) nach Anspruch 11, **dadurch gekennzeichnet, dass** die Düse (3), die Höcker (33), die Verbindungszweige (34) und die Verbindungsklötze (32) aus einem gleichen Stück gebildet sind.

13. Vorrichtung (1) nach Anspruch 12, **dadurch gekennzeichnet, dass** das Stück aus Polycarbonat hergestellt ist.
